# EUROPEAN PATENT SPECIFICATION

(11) **EP 3 472 274 B1**
(45) Date of publication and mention of the grant of the patent: **07.08.2024**
(21) Application number: 17733273.1
(22) Date of filing: 14.06.2017
(51) Int. Cl.: C10M 129/10, C10M 169/04, C07C 37/14, C07C 39/06, C10M 145/24, C10M 149/12, C10M 159/16, C10M 133/14

(54) **LUBRICATING COMPOSITIONS CONTAINING A POLYISOBUTYLENE-SUBSTITUTED PHENOL**
SCHMIERÖLZUSAMMENSETZUNGEN MIT EINEM POLYISOBUTYLEN-SUBSTITUIERTEM PHENOL
COMPOSITIONS LUBRIFIANTES CONTENANT UN PHÉNOL SUBSTITUÉ PAR UN POLYISOBUTYLÈNE

(30) Priority: 17.06.2016 US 201662351483 P; 09.06.2017 US 201762517281 P
(43) Date of publication of application: 24.04.2019
(73) Proprietor: The Lubrizol Corporation, Wickliffe, OH 44092-2298 (US)
(72) Inventor: SAMPLER, Edward P., Belper Derbyshire DE56 1QN (GB); WALKER, Gary M., Belper Derbyshire DE56 1QN (GB); COOK, Stephen J., Belper Derbyshire DE56 1QN (GB); KILSBY, Samuel M., Belper Derbyshire DE56 1QN (GB)
(74) Representative: D Young & Co LLP
(86) International application number: PCT/US2017/037452
(87) International publication number: WO 2017/218657

(56) References cited:
- CA-A- 784 732
- CA-A- 784 732
- DE-A1- 102005 021 093
- DE-A1- 102005 021 093
- DE-A1- 102005 021 093
- DE-A1- 19 948 111
- DE-A1- 19 948 111
- DE-A1- 19 948 111
- GB-A- 785 468
- GB-A- 785 468
- GB-A- 785 468
- US-A- 3 372 116
- US-A- 3 372 116
- US-A- 3 372 116
- US-A- 3 376 221
- US-A- 3 376 221
- US-A- 3 849 084
- US-A- 3 849 084
- US-A- 4 021 419
- US-A- 4 021 419
- US-A- 4 021 419
- US-A- 5 300 701
- US-A- 5 300 701
- US-A- 5 300 701
- US-B2- 7 964 543
- DAVID E. BERGBREITER ET AL: "Synthesis of aryl-substituted polyisobutylenes as precursors for ligands for greener, phase-selectively soluble catalysts", PURE & APPLIED CHEMISTRY, vol. 81, no. 11, 15 January 2009 (2009-01-15), GB, XP055330443, ISSN: 0033-4545, DOI: 10.1351/PAC-CON-08-10-09
- DAVID E. BERGBREITER ET AL: "Synthesis of aryl-substituted polyisobutylenes as precursors for ligands for greener, phase-selectively soluble catalysts", PURE & APPLIED CHEMISTRY, vol. 81, no. 11, 15 January 2009 (2009-01-15), GB, XP055330443, ISSN: 0033-4545, DOI: 10.1351/PAC-CON-08-10-09
- DAVID E. BERGBREITER ET AL: "Synthesis of aryl-substituted polyisobutylenes as precursors for ligands for greener, phase-selectively soluble catalysts", PURE & APPLIED CHEMISTRY, vol. 81, no. 11, 15 January 2009 (2009-01-15), GB, XP055330443, ISSN: 0033-4545, DOI: 10.1351/PAC-CON-08-10-09

## Description

### BACKGROUND

The invention relates generally to a lubricating composition comprising a polyisobutylene-substituted phenol.

Derivatives of phenols, such as phenol-based detergents are known. These include, for example, phenates based on phenolic monomers linked with sulfur bridges or alkylene bridges such as methylene linkages derived from formaldehyde. The phenolic monomers themselves are typically substituted with an aliphatic hydrocarbyl group to provide a measure of oil solubility. The hydrocarbyl groups may be alkyl groups, such as dodecylphenol (or propylene tetramer-substituted phenol). Basic sulfurized polyvalent metal phenates are described, for example, in U.S. Pat. No. 2,680,096, to Walker et al., issued June 1, 1954; and U.S. Pat. No. 3,372,116, to Meinhardt, issued March 6, 1968.

Recently, however, certain alkylphenols and products prepared from them have come under increased scrutiny due to their classification as potentially harmful materials. In particular, alkylphenol detergents which are based on oligomers of C₁₂ alkyl phenols may contain residual monomeric C₁₂ alkyl phenol species. These detergents include those derived from para-dodecyl phenol. There is interest, therefore, in developing alkyl-substituted phenate detergents, for uses in lubricants, fuels, and as industrial additives, which reduce or eliminate the need for dodecylphenol-based compounds.

There have been several efforts to prepare phenol derived detergents that do not contain Cₙ alkyl phenols. U.S. Pat. No. 7,435,709, to Stonebraker, et al., issued October 14, 2008, discloses a linear alkylphenol-derived detergent which is a salt of a reaction product of an olefin having at least 10 carbon atoms and a hydroxyaromatic compound. Greater than 90 mole % of the olefin is a linear C₂₀-C₃₀ n-alpha olefin, less than 10 mole % of the olefin is a linear olefin of less than 20 carbon atoms, and less than 5 mole %of the olefin is a branched chain olefin of 18 carbons or less.

U.S. Pub. No. 2011/0190185, to Sinquin, et al, published August 4, 2011, discloses an overbased salt of an oligomerized alkylhydroxyaromatic compound. The alkyl group is derived from an olefin mixture comprising propylene oligomers having an initial boiling point of at least 195 °C and a final boiling point of greater than 325 °C. The propylene oligomers may contain a distribution of carbon atoms that include at least 50 wt. % C₁₄ to C₂₀ carbon atoms.

U.S. Pub. No. 2011/0124539, to Sinquin, et al, published May 26, 2011, discloses an overbased, sulfurized salt of an alkylated hydroxyaromatic compound. The alkyl substituent is a residue of at least one isomerized olefin having from 15 to 99 wt. % branching. The hydroxyaromatic compound may be a phenol, cresol, xylenol, or mixture thereof.

U.S. Pub. No. 2011/0118160, to Campbell, et al., published May 19, 2011, discloses an alkylated hydroxyaromatic compound prepared by reacting a hydroxyaromatic compound with at least one branched olefinic propylene oligomer having from 20 to 80 carbon atoms. Suitable hydroxyaromatic compounds include phenol, catechol, resorcinol, hydroquinone, pyrogallol, and cresol.

U.S. Pub. No. 2010/0029529, to Campbell, et al., published February 4, 2010, discloses an overbased salt of an oligomerized alkylhydroxyaromatic compound. The alkyl group is derived from an olefin mixture comprising propylene oligomers having an initial boing point of at least 195°C and a final boiling point of no more than 325 °C.

U.S. Pub. No. 2008/0269351, to Campbell, et al., published October 30, 2008, discloses an alkylated hydroxyaromatic compound prepared by reacting a hydroxyaromatic compound with a branched olefinic oligomer having from 20 to 80 carbon atoms.

U.S. Pat. No. 6,310,009, to Carrick, et al., issued October 30, 2001, discloses bridged phenolic compounds, each phenol group being substituted with an alkyl group of 1 to 60 carbon atoms, e.g., 9 to 18 carbon atoms. Patents or Patent Applications US 4,021,419; US 3,372,116; DE 199 48 111; GB 785,468; DE 10 2005 021 093; US 7,964,543; US 3,376,221; and CA 784 732 disclose polyisobutylene-substituted phenol derivatives, such as Mannich adducts or condensation products of polyisobutylene-substituted phenols, and polyisobutylene-substituted metal phenates.

There remains a need for a phenolic material with appropriate oil solubility, viscosity performance, and detergency (characteristic of moderate chain length alkyl groups) but free from or substantially free from C₁₂ alkyl phenol moieties.

### BRIEF DESCRIPTION

The present invention includes a lubricating composition comprising
(I) an oil of lubricating viscosity and
(II) a polyisobutylene-substituted phenol having the formula: 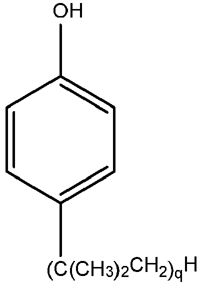 where q is from 3 to 10 and comprises the reaction product of a phenol with a polyisobutylene having a number average molecular weight of 300 to 500.

The present invention also comprises a method of forming a lubricating composition comprising combining the polyisobutylene-substituted phenol as described herein with an oil of lubricating viscosity.

The present invention also comprises the use of the lubricating composition as described herein for lubricating a mechanical device.

### DETAILED DESCRIPTION

The present invention relates to a lubricating composition comprising a polyisobutylene-substituted phenol, a method of forming said lubricating composition and a use of said lubricating composition for lubricating a mechanical device.

Unless otherwise stated, molecular weight herein is reported as number average molecular weight, in Daltons, and is measured by Gel Permeation Chromatography (GPC) using a polyisobutylene standard.

The polyisobutylene-substituted phenol compound has the formula: 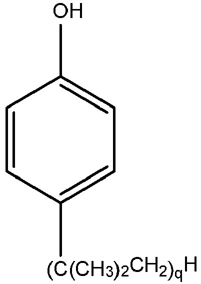 where q is from 3 to 10 and comprises the reaction product of a phenol with a polyisobutylene having a number average molecular weight of 300 to 500. In one embodiment, q may be 4, 5, 6, 7, 8, 9, 10, or mixtures of one or more of these. In one particular embodiment, the polyisobutylene-substituted phenol compound comprises the reaction product of a phenol, with a polyisobutylene having a number average molecular weight of 300 to 400.

The term "substantially free" as it refers to the exemplary compounds described herein, means that less than 0.01 mol. %, or less than 0.001 mol. %, or less than 0.0001 mol. % of the polyisobutylene-substituted hydroxyaromatic compound in the lubricating composition is substituted with one or more C₁₂ alkyl groups. In one embodiment, the lubricating compound contains less than 0.5 wt. %, or less than 0.1 wt. %, or less than 0.01 wt. %, or less than 0.001 wt. %, of C₁₂ alkyl hydroxyaromatic compounds.

### Formation of the Polyisobutylene-Substituted Phenol Compound

The polyisobutylene-substituted phenol intermediate compound of the present invention may be formed by reaction of a phenol with a poly(branched alkene). Substitution occurs primarily at the para position, although minor amounts of *ortho-* and/or *meta-* substitution may occur. The ratio of poly(branched alkene) to phenol may be from 1:1 to 1:6, such as at least 1:2. An excess of phenol helps to ensure primarily mono-substitution of the poly(branched alkene).

In one embodiment, the poly(branched alkene) may be a polysobutylene. For example, the polyisobutylene may be a highly reactive polyisobutylene, which differs from the polyisobutylenes having low reactivity through the content of terminally arranged ethylenic double bonds. Suitable highly reactive polyisobutylenes are, for example, polyisobutylenes which have more than 70, in particular more than 80, especially more than 85, mol %, based on the polyisobutylene macromolecules, of vinylidene double bonds. Particularly preferred polyisobutylenes are those which have uniform polymer backbones. In particular, those polymers which are composed of at least 85, preferably at least 90, particularly preferably at least 95, % by weight of isobutene units have uniform polymer backbones. In one useful embodiment, a highly reactive polyisobutylene used for the alkylation of a phenol as described herein will have a number average molecular weight of at least 300, and up to 500, or up to 400, or even up to 360. In one embodiment, the polyisobutylene has a number average molecular weight of 300 to 400. In some embodiments, useful polyisobutylenes may have a polydispersity of greater than 1.5, for example 1.5 to 3.0. Polydispersity is understood as meaning the quotient of weight average molecular weight M_{W} and number average molecular weight M_{N}.

In another embodiment, the polyisobutylene useful in this invention is made in the absence of a chain transfer agent. CTAs are known in literature. For example, J. P. Kennedy et al, *Carbocationic Polymerization* (1982), page 229, John Wiley & Sons, New York, list several chain transfer agents and their transfer coefficients. Particularly suitable CTAs for the present reaction are selected from the group consisting of 2,4,4-Trimethyl-1-pentene ("α-DIB"), 2.4.4.-Trimethyl-2-pentene ("β-DIB"), 2-ethyl-1-hexene, 2-methyl-1-pentene and mixtures thereof. Of these, α-DIB, β-DIB, or mixtures thereof are preferred. The structures of α-DIB and β-DIB are shown below: 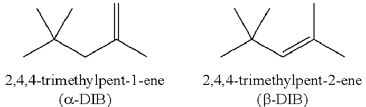 Other CTAs may include 2-octene; 2,5-dimethyl-2,4-hexadiene; cyclohexadiene; isoprene; piperylene and vinylcyclohexane. In general, the chain transfer agent in an olefinic molecule with a molecular weight higher than isobutene and lower than the low molecular weight polymer product produced in accordance with the invention. The CTA is readily detectable by GPC in the product composition by GPC.

In one useful embodiment, the polyisobutylene useful in the present invention is prepared without the use of a chain transfer agent as described above.

To form the polyisobutylene-substituted phenol compound, a phenol, is reacted (alkylated) with a polyisobutylene as described above.

In the alkylation, the phenol is usually used in excess. For example, about a 1.1- to 6-fold, preferably 1.6- to 5-fold, excess, such as a 2-fold or a 4-fold excess, of the phenol is suitable.

In one embodiment of the present process, the phenol is used in excess in the preparation of the polyisobutenylphenol and, after the end of the reaction, the reaction mixture is freed from excess phenol by extraction with solvents, preferably polar solvents, such as water or C₁-C₆-alkanols or mixtures thereof, by stripping, i.e. by passing steam through or, if required, heating of gases, e.g. nitrogen, or by distillation.

The process to prepare the intermediate may be carried out a reaction temperature of 1°C to 52°C, or 5°C to 20°C, or 10°C to 15°C.

Suitable alkylation catalysts are known to a person skilled in the art. For example, protic acids, such as sulfuric acid, phosphoric acid and organic sulfonic acids, e.g. trifluoromethanesulfonic acid, Lewis acids, such as aluminum trihalides, e.g. aluminum trichloride or aluminum tribromide, boron trihalides, e.g. boron trifluoride and boron trichloride, tin halides, e.g. tin tetrachloride, titanium halides, e.g. titanium tetrabromide and titanium tetrachloride, and iron halides, e.g. iron trichloride and iron tribromide, are suitable. Adducts of boron trihalides, in particular boron trifluoride, with electron donors such as alcohols, in particular C₁-C₆-alkanols or phenols, or ethers are preferred. Boron trifluoride etherate is particularly preferred.

The alkylation may be carried out in a liquid medium. For this purpose, the phenol is preferably dissolved in one of the reactants and/or in a solvent, if necessary with heating. In a preferred embodiment, the alkylation is preferably carried out by first melting the phenol or the substituted phenol by supplying heat and then adding a suitable solvent and/or the alkylation catalyst, in particular the boron trihalide adduct. The liquid mixture is then brought to a suitable reaction temperature. In a further preferred embodiment the phenol is first melted and the polyisobutylene and, if required, suitable solvent are added. The liquid mixture thus obtained can be brought to a suitable reaction temperature and the alkylation catalyst can then be added.

Suitable solvents for carrying out this reaction are, for example, hydrocarbons, including but not limited to pentane, hexane and heptane or hydrocarbon mixtures, such as petroleum naphthas having boiling ranges from 35 to 100° C., dialkyl ethers, in particular diethyl ether, and halogenated hydrocarbons, such as dichloromethane or trichloromethane, and mixtures of these solvents.

The reaction may be initiated by adding the catalyst or one of the two reactants, phenol or polyisobutylene. The addition of the component initiating the reaction may be done over a period of from 5 to 300, or even from 10 to 200, or even from 15 to 180, minutes. In some embodiments, it is useful to not have the temperature of the reaction mixture exceeding the above-mentioned temperature ranges. After the end of the addition, the reaction mixture is allowed to continue reacting for preferably from 30 minutes to 24 hours, in particular from 60 minutes to 16 hours, at below 30° C. The reaction conditions are preferably chosen so that at least 85%, in particular at least 90%, particularly preferably at least 95%, of the polyisobutenylphenol form. The polyisobutenyl-substituted phenols thus obtained preferably comprise (where the aromatic hydroxy compound used as the starting material allows) more than 85%, in particular more than 90%, and particularly preferably more than 95%, of isomers whose polyisobutenyl radical is para to the hydroxyl group of the phenol.

Optionally the solvent is removed before further reaction to form any derivatives of the polyisobutylene-substituted phenol compound. The reaction mixture may then be neutralized with calcium hydroxide, followed by addition of diatomaceous earth and ammonia hydroxide to remove residual catalyst. The reaction mixture is filtered and the filtrate heated under vacuum to remove volatiles by distillation

When the phenol used for the alkylation as described above allows multiple alkylations, the reaction may be carried out in such a way that the polyisobutenylphenols obtained include little if any product more than monoalkylated by the polyisobutylene.

The polyisobutylene-substituted phenol in accordance with the present invention has the formula: 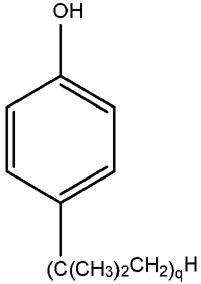 where q is from 3 to 10, may be formed by reacting phenol with a polyisobutylene, optionally in the presence of a base catalyst.

Reaction Scheme 1 illustrates the reaction: 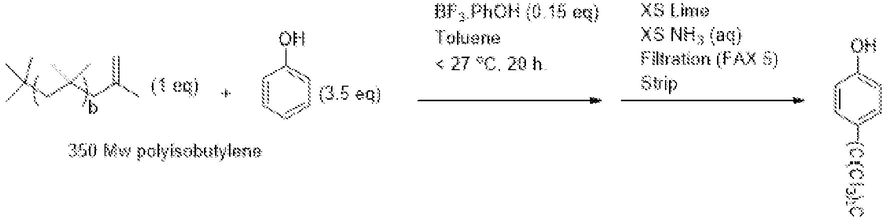 where b is, for example, from 2-8 and q is, for example 4-8.

### Lubricating Compositions

The polyisobutylene-substitued phenol may be present in the lubricating composition at a concentration of at least 0.1 wt. % and may be up to 20 wt. %. For example, the concentration of the compound may be at least 0.25 wt. %, or at least 0.5 wt. %, or at least 1 wt. %, or at least 5 wt. %, or at least 10 wt. %, or at least 15 wt. % of the lubricating composition. The compound may also be present in a concentrate, alone or with other additives and with a lesser amount of oil. In a concentrate, the amount of the compound may be at least 2, or at least 3 times the concentration in the lubricating composition.

In addition to the polyisobutylene-substituted phenol compound, the exemplary lubricating composition includes an oil of lubricating viscosity and optionally one or more additional performance additives suited to providing the performance properties of a fully formulated lubricating compositions. The polyisobutylene-substituted phenol and derivatives thereof of the present invention may be useful in a variety of lubricating applications, including but not limited to engine oils, hydraulic oils, industrial gear oils, metalworking fluids, refrigerant lubricants, driveline fluids, and transmission fluids. Examples of these additional performance additives include (overbased) detergents, viscosity modifiers, friction modifiers, antioxidants, dispersants, antiwear/antiscuffing agents, metal deactivators, extreme pressure agents, foam inhibitors, demulsifiers, pour point depressants, corrosion inhibitors, and seal swelling agents, which may be used singly or in combination. Oil of lubricating viscosity

The lubricating composition may include the oil of lubricating viscosity as a minor or major component thereof, such as at least 5 wt. %, or at least 10 wt. %, or at least 20 wt. %, or at least 30 wt. %, or at least 40 wt. %, or at least 60 wt. %, or at least 80 wt. % of the lubricating composition.

Suitable oils include natural and synthetic oils, oil derived from hydrocracking, hydrogenation, and hydrofinishing, unrefined, refined, re-refined oils or mixtures thereof. Unrefined, refined and re-refined oils, and natural and synthetic oils are described, for example, in WO2008/147704 and US Pub. No. 2010/197536. Synthetic oils may also be produced by Fischer-Tropsch reactions and typically may be hydroisomerized Fischer-Tropsch hydrocarbons or waxes. Oils may be prepared by a Fischer-Tropsch gas-to-liquid synthetic procedure as well as other gas-to-liquid procedures.

Oils of lubricating viscosity may also be defined as specified in April 2008 version of "Appendix E - API Base Oil Interchangeability Guidelines for Passenger Car Motor Oils and Diesel Engine Oils", section 1.3 Sub-heading 1.3. "Base Stock Categories". The API Guidelines are also summarized in US Pat. No. 7,285,516. The five base oil groups are as follows: Group I (sulfur content >0.03 wt. %, and/or <90 wt. % saturates, viscosity index 80-120); Group II (sulfur content 0.03 wt. %, and ≥90 wt. % saturates, viscosity index 80-120); Group III (sulfur content 0.03 wt. %, and ≥90 wt. % saturates, viscosity index ≥120); Group IV (all polyalphaolefins (PAOs)); and Group V (all others not included in Groups I, II, III, or IV). The exemplary oil of lubricating viscosity includes an API Group I, Group II, Group III, Group IV, Group V oil, or mixtures thereof. In some embodiments, the oil of lubricating viscosity is an API Group I, Group II, Group III, or Group IV oil, or mixtures thereof. In some embodiments, the oil of lubricating viscosity is an API Group I, Group II, or Group III oil, or mixture thereof. In one embodiment the oil of lubricating viscosity may be an API Group II, Group III mineral oil, a Group IV synthetic oil, or mixture thereof. In some embodiments, at least 5 wt. %, or at least 10 wt. %, or at least 20 wt. %, or at least 40 wt. % of the lubricating composition is a polyalphaolefin (Group IV).

The oil of lubricating viscosity may have a kinematic viscosity of up to 30 mm²/s or up to 25 mm²/s (cSt) at 100 °C and can be at least 12 mm²/s at 100 °C, and in other embodiments at least 15 mm²/s. As used herein, kinematic viscosity is determined at 100 °C by ASTM D445-14, "Standard Test Method for Kinematic Viscosity of Transparent and Opaque Liquids (and Calculation of Dynamic Viscosity)," ASTM International, West Conshohocken, PA, 2003, DOI: 10.1520/D0445-14 and may be referred to as KV_100.

In certain embodiments, the lubricating composition may contain synthetic ester base fluids. Synthetic esters may have a kinematic viscosity measured at 100°C of 2.5 mm²/s to 30 mm²/s. In one embodiment, the lubricating composition comprises less than 50 wt. % of a synthetic ester base fluid with a KV_100 of at least 5.5 mm²/s, or at least 6 mm²/s, or at least 8 mm²/s.

Exemplary synthetic oils include poly-alpha olefins, polyesters, polyacrylates, and poly-methacrylates, and co-polymers thereof. Example synthetic esters include esters of a dicarboxylic acid (e.g., selected from phthalic acid, succinic acid, alkyl succinic acids, alkenyl succinic acids, maleic acid, azelaic acid, suberic acid, sebacic acid, fumaric acid, adipic acid, linoleic acid dimer, malonic acid, alkyl malonic acids, and alkenyl malonic acids) with an alcohol (e.g., selected from butyl alcohol, hexyl alcohol, dodecyl alcohol, 2-ethylhexyl alcohol, ethylene glycol, diethylene glycol monoether, and propylene glycol). Specific examples of these esters include dibutyl adipate, di(2-ethylhexyl) sebacate, di-n-hexyl fumarate, dioctyl sebacate, diisooctyl azelate, diisodecyl azelate, dioctyl phthalate, didecyl phthalate, dieicosyl sebacate, the 2-ethylhexyl diester of linoleic acid dimer, and the complex ester formed by reacting one mole of sebacic acid with two moles of tetraethylene glycol and two moles of 2-ethylhexanoic acid.

Esters useful as synthetic oils also include those made from C₅ to C₁₂ monocarboxylic acids and polyols and from polyol ethers such as neopentyl glycol, trimethylolpropane, pentaerythritol, dipentaerythritol, and tripentaerythritol. Esters can also be monoesters, such as are available under the trade name Priolube 1976^{™} (C₁₈-alkyl-COO-C₂₀ alkyl).

Synthetic ester base oils may be present in the lubricating composition of the invention in an amount less than 50 wt. % of the composition, or less than 40 weight %, or less than 35 weight %, or less than 28 weight %, or less than 21 weight %, or less than 17 weight %, or less than 10 weight %, or less than 5 weight % of the composition. In one embodiment, the lubricating composition of the invention is free of, or substantially free of, a synthetic ester base fluid having a KV_100 of at least 5.5 mm²/s.

Example natural oils include animal and vegetable oils, such as long chain fatty acid esters. Examples include linseed oil, sunflower oil, sesame seed oil, beef tallow oil, lard oil, palm oil, castor oil, cottonseed oil, corn oil, peanut oil, soybean oil, olive oil, whale oil, menhaden oil, sardine oil, coconut oil, palm kernel oil, babassu oil, rape oil, and soya oil.

The amount of the oil of lubricating viscosity present is typically the balance remaining after subtracting from 100 weight % the sum of the amount of the exemplary amino-carboxylate compound and the other performance additives.

### Other Additives

### Detergents

The lubricating composition optionally further includes at least one detergent, other than the exemplary compound. Exemplary detergents useful herein include overbased metal-containing detergents. The metal of the metal-containing detergent may be zinc, sodium, calcium, barium, or magnesium. The overbased metal-containing detergent may be chosen from sulfonates, non-sulfur containing phenates, sulfur containing phenates, salixarates, salicylates, and mixtures thereof, or borated equivalents thereof. The overbased detergent may be borated with a borating agent such as boric acid.

The overbased metal-containing detergent may also include "hybrid" detergents formed with mixed surfactant systems including phenate and/or sulfonate components, e.g., phenate/salicylates, sulfonate/phenates, sulfonate/salicylates, sulfonates/phenates/salicylates, as described, for example, in U.S. Pat. Nos. 6,429,178; 6,429,179; 6,153,565; and 6,281,179. Where a hybrid sulfonate/phenate detergent is employed, the hybrid detergent can be considered equivalent to amounts of distinct phenate and sulfonate detergents introducing like amounts of phenate and sulfonate soaps, respectively.

Example overbased metal-containing detergents include zinc, sodium, calcium and magnesium salts of sulfonates, phenates (including sulfur-containing and non-sulfur containing phenates), salixarates and salicylates. Such overbased sulfonates, salixarates, phenates and salicylates may have a total base number of 120 to 700, or 250 to 600, or 300 to 500 (on an oil free basis).

The overbased sulfonate detergent may have a metal ratio of 12 to less than 20, or 12 to 18, or 20 to 30, or 22 to 25.

Typically, an overbased metal-containing detergent may be a zinc, sodium, calcium or magnesium salt of a sulfonate, a phenate, sulfur containing phenate, salixarate or salicylate. Overbased sulfonates, salixarates, phenates and salicylates typically have a total base number of 120 to 700 TBN. Overbased sulfonates typically have a total base number of 120 to 700, or 250 to 600, or 300 to 500 (on an oil free basis).

The overbased sulfonate detergent may have a metal ratio of 12 to less than 20, or 12 to 18, or 20 to 30, or 22 to 25.

Example sulfonate detergents include linear and branched alkylbenzene sulfonate detergents, and mixtures thereof, which may have a metal ratio of at least 8, as described, for example, in U.S. Pub. No. 2005065045. Linear alkyl benzenes may have the benzene ring attached anywhere on the linear chain, usually at the 2, 3, or 4 position, or be mixtures thereof. Linear alkylbenzene sulfonate detergents may be particularly useful for assisting in improving fuel economy.

In one embodiment, the alkylbenzene sulfonate detergent may be a branched alkylbenzene sulfonate, a linear alkylbenzene sulfonate, or mixtures thereof.

In one embodiment, the lubricating composition may be free of linear alkylbenzene sulfonate detergent. The sulfonate detergent may be a metal salt of one or more oil-soluble alkyl toluene sulfonate compounds as disclosed in U.S. Pub. No. 20080119378.

The lubricating composition may include at least 0.01 wt. % or at least 0.1 wt. % of the detergent other than the exemplary compound, and in some embodiments, up to 2 wt. %, or up to 1 wt. % detergent.

### Antioxidants

The lubricating composition optionally further includes at least one antioxidant. Exemplary antioxidants useful herein include phenolic and aminic antioxidants, such as diarylamines, alkylated diarylamines, hindered phenols, and mixtures thereof. The diarylamine or alkylated diarylamine may be a phenyl-α-naphthylamine (PANA), an alkylated diphenylamine, an alkylated phenylnapthylamine, or mixture thereof. Example alkylated diphenylamines include dinonyl diphenylamine, nonyl diphenylamine, octyl diphenylamine, dioctyl diphenylamine, didecyl diphenylamine, decyl diphenylamine, and mixtures thereof. Example alkylated diarylamines include octyl, dioctyl, nonyl, dinonyl, decyl and didecyl phenylnapthylamines. Hindered phenol antioxidants often contain a secondary butyl and/or a tertiary butyl group as a steric hindering group. The phenol group may be further substituted with a hydrocarbyl group (e.g., a linear or branched alkyl) and/or a bridging group linking to a second aromatic group. Examples of suitable hindered phenol antioxidants include 2,6-di-tertbutylphenol, 4-methyl-2,6-di-tert-butylphenol, 4-ethyl-2,6-di-tert-butylphenol, 4-propyl-2,6-di-tert-butylphenol, 4-butyl-2,6-di-tert-butylphenol, and 4-dodecyl-2,6-di-tert-butylphenol. In one embodiment, the hindered phenol antioxidant may be an ester, such as those described in U.S. Pat. No. 6,559,105. One such hindered phenol ester is sold as Irganox^{™} L-135, obtainable from Ciba.

When present, the lubricating composition may include at least 0.1 wt. % or at least 0.5 wt. %, or at least 1 wt. % antioxidant, and in some embodiments, up to 3 wt. %, or up to 2.75 wt. %, or up to 2.5 wt. % antioxidant.

### Dispersants

The lubricating composition optionally further includes at least one dispersant, other than the exemplary compound. Exemplary dispersants include succinimide dispersants, Mannich dispersants, succinamide dispersants, and polyolefin succinic acid esters, amides, and ester-amides, and mixtures thereof. The succinimide dispersant, where present, may be as described above for the succinimides described as useful for cation M.

The succinimide dispersant may be derived from an aliphatic polyamine, or mixtures thereof. The aliphatic polyamine may be an ethylenepolyamine, a propylenepolyamine, a butylenepolyamine, or a mixture thereof. In one embodiment the aliphatic polyamine may be an ethylenepolyamine. In one embodiment the aliphatic polyamine may be chosen from ethylenediamine, diethylenetriamine, triethylenetetramine, tetraethylenepentamine, pentaethylenehexamine, polyamine still bottoms, and mixtures thereof.

In one embodiment, the dispersant may be a polyolefin succinic acid ester, amide, or ester-amide. A polyolefin succinic acid ester-amide may be a polyisobutylene succinic acid reacted with an alcohol (such as pentaerythritol) and a polyamine as described above. Example polyolefin succinic acid esters include polyisobutylene succinic acid esters of pentaerythritol and mixture thereof.

The dispersant may be an N-substituted long chain alkenyl succinimide. An example of an N-substituted long chain alkenyl succinimide is polyisobutylene succinimide. Typically the polyisobutylene from which polyisobutylene succinic anhydride is derived has a number average molecular weight of 350 to 5000, or 550 to 3000 or 750 to 2500. Succinimide dispersants and their preparation are disclosed, for example, in US Pat. Nos. 3,172,892, 3,219,666, 3,316,177, 3,340,281, 3,351,552, 3,381,022, 3,433,744, 3,444,170, 3,467,668, 3,501,405, 3,542,680, 3,576,743, 3,632,511, 4,234,435, Re 26,433, and 6,165,235, and 7,238,650 and EP Patent Application 0 355 895 A.

The succinimide dispersant may comprise a polyisobutylene succinimide, wherein the polyisobutylene from which polyisobutylene succinimide is derived has a number average molecular weight of 350 to 5000, or 750 to 2500.

The exemplary dispersants may also be post-treated by conventional methods by a reaction with any of a variety of agents. Among these are boron compounds (such as boric acid), urea, thiourea, dimercaptothiadiazoles, carbon disulfide, aldehydes, ketones, carboxylic acids, such as terephthalic acid, hydrocarbon-substituted succinic anhydrides, maleic anhydride, nitriles, epoxides, and phosphorus compounds. In one embodiment the post-treated dispersant is borated. In one embodiment the post-treated dispersant is reacted with dimercaptothiadiazoles. In one embodiment the post-treated dispersant is reacted with phosphoric or phosphorous acid. In one embodiment the post-treated dispersant is reacted with terephthalic acid and boric acid (as described in U.S. Pub. No. 2009/0054278.

When present, the lubricating composition may include at least 0.01 wt. %, or at least 0.1 wt. %, or at least 0.5 wt. %, or at least 1 wt. % of other dispersant(s), and in some embodiments, up to 20 wt. %, or up to 15 wt. %, or up to 10 wt. %, or up to 6 wt. % or up to 3 wt. % dispersant.

### Anti-wear Agents

The lubricating composition optionally further includes at least one antiwear agent. Examples of suitable antiwear agents suitable for use herein include titanium compounds, tartrates, tartrimides, oil soluble amine salts of phosphorus compounds, sulfurized olefins, metal dihydrocarbyldithiophosphates (such as zinc dialkyldithiophosphates), phosphites (such as dibutyl phosphite), phosphonates, thiocarbamate-containing compounds, such as thiocarbamate esters, thiocarbamate amides, thiocarbamic ethers, alkylene-coupled thiocarbamates, and bis(S-alkyldithiocarbamyl) disulfides. The antiwear agent may in one embodiment include a tartrate, or tartrimide as described in U.S. Pub. Nos. 2006/0079413; 2006/0183647; and 2010/0081592. The tartrate or tartrimide may contain alkyl-ester groups, where the sum of carbon atoms on the alkyl groups is at least 8. The antiwear agent may, in one embodiment, include a citrate as is disclosed in US Pub. No. 20050198894.

The lubricating composition may in one embodiment further include a phosphorus-containing antiwear agent. Example phosphorus-containing antiwear agents include zinc dialkyldithiophosphates, phosphites, phosphates, phosphonates, and ammonium phosphate salts, and mixtures thereof.

When present, the lubricating composition may include at least 0.01 wt. %, or at least 0.1 wt. %, or at least 0.5 wt. % antiwear agent, and in some embodiments, up to 3 wt. %, or up to 1.5 wt. %, or up to 0.9 wt. antiwear agent.

### Oil-soluble Titanium Compounds

The lubricating composition may include one or more oil-soluble titanium compounds, which may function as antiwear agents, friction modifiers, antioxidants, deposit control additives, or more than one of these functions. Example oil-soluble titanium compounds are disclosed in U.S. Pat. No. 7,727,943 and U.S. Pub. No. 2006/0014651. Example oil soluble titanium compounds include titanium (IV) alkoxides, such as titanium (IV) isopropoxide and titanium (IV) 2 ethylhexoxide. Such alkoxides may be formed from a monohydric alcohol, a vicinal 1,2-diol, a polyol, or mixture thereof. The monohydric alkoxides may have 2 to 16, or 3 to 10 carbon atoms. In one embodiment, the titanium compound comprises the alkoxide of a vicinal 1,2-diol or polyol. 1,2-vicinal diols include fatty acid mono-esters of glycerol, where the fatty acid may be, for example, oleic acid. Other example oil soluble titanium compounds include titanium carboxylates, such as titanium neodecanoate.

When present in the lubricating composition, the amount of oil-soluble titanium compounds is included as part of the antiwear agent.

### Extreme Pressure (EP) agents

The lubricating composition may include an extreme pressure agent. Example extreme pressure agents that are soluble in the oil include sulfur- and chlorosulfur-containing EP agents, dimercaptothiadiazole or CS₂ derivatives of dispersants (typically succinimide dispersants), derivative of chlorinated hydrocarbon EP agents and phosphorus EP agents. Examples of such EP agents include chlorinated wax; sulfurized olefins (such as sulfurized isobutylene), hydrocarbyl-substituted 2,5-dimercapto-1,3,4-thiadiazoles and oligomers thereof, organic sulfides and polysulfides, such as dibenzyldisulfide, bis-(chlorobenzyl) disulfide, dibutyl tetrasulfide, sulfurized methyl ester of oleic acid, sulfurized alkylphenol, sulfurized dipentene, sulfurized terpene, and sulfurized Diels-Alder adducts; phosphosulfurized hydrocarbons such as the reaction product of phosphorus sulfide with turpentine or methyl oleate; phosphorus esters, such as dihydrocarbon and trihydrocarbon phosphites, e.g., dibutyl phosphite, diheptyl phosphite, dicyclohexyl phosphite, pentylphenyl phosphite; dipentylphenyl phosphite, tridecyl phosphite, distearyl phosphite and polypropylene substituted phenol phosphite; metal thiocarbamates, such as zinc dioctyldithiocarbamate and barium heptylphenol diacid; amine salts of alkyl and dialkylphosphoric acids or derivatives including, for example, the amine salt of a reaction product of a dialkyldithiophosphoric acid with propylene oxide and subsequently followed by a further reaction with P₂O₅; and mixtures thereof. Some useful extreme pressure agents are described in US Pat. No. 3,197,405.

When present, the lubricating composition may include at least 0.01 wt. %, or at least 0.1 wt. %, or at least 0.5 wt. % extreme pressure agent, and in some embodiments, up to 3 wt. %, or up to 1.5 wt. %, or up to 0.9 wt. % of the extreme pressure agent.

### Foam Inhibitors

The lubricating composition may include a foam inhibitor. Foam inhibitors that may be useful in the lubricant composition include polysiloxanes; copolymers of ethyl acrylate and 2-ethylhexylacrylate and optionally vinyl acetate; demulsifiers including fluorinated polysiloxanes, trialkyl phosphates, polyethylene glycols, polyethylene oxides, polypropylene oxides and (ethylene oxide-propylene oxide) polymers.

### Viscosity Modifiers

The lubricating composition may include a viscosity modifier. Viscosity modifiers (also sometimes referred to as viscosity index improvers or viscosity improvers) useful in the lubricant composition are usually polymers, including polyisobutylenes, polymethacrylates (PMA) and polymethacrylic acid esters, diene polymers, polyalkylstyrenes, esterified styrene-maleic anhydride copolymers, hydrogenated alkenylarene-conjugated diene copolymers and polyolefins also referred to as olefin copolymer or OCP. PMA's are prepared from mixtures of methacrylate monomers having different alkyl groups. The alkyl groups may be either straight chain or branched chain groups containing from 1 to 18 carbon atoms. Most PMA's are viscosity modifiers as well as pour point depressants. In one embodiment, the viscosity modifier is a polyolefin comprising ethylene and one or more higher olefin, such as propylene.

When present, the lubricating composition may include at least 0.01 wt. %, or at least 0.1 wt. %, or at least 0.3 wt. %, or at least 0.5 wt. % polymeric viscosity modifiers, and in some embodiments, up to 10 wt. %, or up to 5 wt. %, or up to 2.5 wt. % polymeric viscosity modifiers.

### Corrosion Inhibitors and Metal Deactivators

The lubricating composition may include a corrosion inhibitor. Corrosion inhibitors/metal deactivators that may be useful in the exemplary lubricating composition include fatty amines, octylamine octanoate, condensation products of dodecenyl succinic acid or anhydride, and a fatty acid such as oleic acid with a polyamine, derivatives of benzotriazoles (e.g., tolyltriazole), 1,2,4-triazoles, benzimidazoles, 2-alkyldithiobenzimidazoles and 2-alkyldithiobenzothiazoles.

### Pour Point Depressants

The lubricating composition may include a pour point depressant. Pour point depressants that may be useful in the exemplary lubricating composition include polyalphaolefins, esters of maleic anhydride-styrene copolymers, polymethacrylates, polyacrylates, and polyacrylamides.

### Friction Modifiers

The lubricating composition may include a friction modifier. Friction modifiers that may be useful in the exemplary lubricating composition include fatty acid derivatives such as amines, esters, epoxides, fatty imidazolines, condensation products of carboxylic acids and polyalkylene-polyamines and amine salts of alkylphosphoric acids. The friction modifier may be an ash-free friction modifier. Such friction modifiers are those which typically not produce any sulfated ash when subjected to the conditions of ASTM D 874. An additive is referred to as "non-metal containing" if it does not contribute metal content to the lubricant composition. As used herein the term "fatty alkyl" or "fatty" in relation to friction modifiers means a carbon chain having 8 to 30 carbon atoms, typically a straight carbon chain.

In one embodiment, the ash-free friction modifier may be represented by the formula: 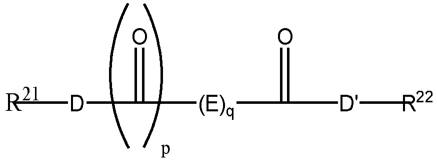 where, D and D' are independently selected from -O-, >NH, >NR²³, an imide group formed by taking together both D and D' groups and forming a R²¹-N< group between two >C=O groups; E is selected from -R²⁴-O-R²⁵-, >CH₂, >CHR²⁶, >CR²⁶R²⁷, >C(OH)(CO₂R²²), >C(CO₂R²²)₂, and >CHOR²⁸; where R²⁴ and R²⁵ are independently selected from >CH₂, >CHR²⁶, >CR²⁶R²⁷, >C(OH)(CO₂R²²), and >CHOR²⁸; q is 0 to 10, with the proviso that when q=1, E is not >CH₂, and when n=2, both Es are not >CH₂; p is 0 or 1; R²¹ is independently hydrogen or a hydrocarbyl group, typically containing 1 to 150 carbon atoms, with the proviso that when R²¹ is hydrogen, p is 0, and q is more than or equal to 1; R²² is a hydrocarbyl group, typically containing 1 to 150 carbon atoms; R²³, R²⁴, R²⁵, R²⁶ and R²⁷ are independently hydrocarbyl groups; and R²⁸ is hydrogen or a hydrocarbyl group, typically containing 1 to 150 carbon atoms, or 4 to 32 carbon atoms, or 8 to 24 carbon atoms. In certain embodiments, the hydrocarbyl groups R²³, R²⁴, and R²⁵, may be linear or predominantly linear alkyl groups.

In certain embodiments, the ash-free friction modifier is a fatty ester, amide, or imide of various hydroxy-carboxylic acids, such as tartaric acid, malic acid lactic acid, glycolic acid, and mandelic acid. Examples of suitable materials include tartaric acid di(2-ethylhexyl) ester (i.e., di(2-ethylhexyl)tartrate), di(C₈-C₁₀)tartrate, di(C₁₂₋₁₅)tartrate, di-oleyltartrate, oleyltartrimide, and oleyl maleimide.

In certain embodiments, the ash-free friction modifier may be chosen from long chain fatty acid derivatives of amines, fatty esters, or fatty epoxides; fatty imidazolines such as condensation products of carboxylic acids and polyalkylene-polyamines; amine salts of alkylphosphoric acids; fatty alkyl tartrates; fatty alkyl tartrimides; fatty alkyl tartramides; fatty phosphonates; fatty phosphites; borated phospholipids, borated fatty epoxides; glycerol esters; borated glycerol esters; fatty amines; alkoxylated fatty amines; borated alkoxylated fatty amines; hydroxyl and polyhydroxy fatty amines including tertiary hydroxy fatty amines; hydroxy alkyl amides; metal salts of fatty acids; metal salts of alkyl salicylates; fatty oxazolines; fatty ethoxylated alcohols; condensation products of carboxylic acids and polyalkylene polyamines; or reaction products from fatty carboxylic acids with guanidine, aminoguanidine, urea, or thiourea and salts thereof.

Friction modifiers may also encompass materials such as sulfurized fatty compounds and olefins, sunflower oil or soybean oil monoester of a polyol and an aliphatic carboxylic acid.

In another embodiment the friction modifier may be a long chain fatty acid ester. In another embodiment the long chain fatty acid ester may be a monoester and in another embodiment the long chain fatty acid ester may be a triglyceride.

The amount of the ash-free friction modifier in a lubricant may be 0.1 to 3 percent by weight (or 0.12 to 1.2 or 0.15 to 0.8 percent by weight). The material may also be present in a concentrate, alone or with other additives and with a lesser amount of oil. In a concentrate, the amount of material may be two to ten times the above concentration amounts.

Molybdenum compounds are also known as friction modifiers. The exemplary molybdenum compound does not contain dithiocarbamate moieties or ligands.

Nitrogen-containing molybdenum materials include molybdenum-amine compounds, as described in U.S. Pat. No. 6,329,327, and organomolybdenum compounds made from the reaction of a molybdenum source, fatty oil, and a diamine as described in U.S. Pat. No. 6,914,037. Other molybdenum compounds are disclosed in U.S. Pub. No. 20080280795. Molybdenum amine compounds may be obtained by reacting a compound containing a hexavalent molybdenum atom with a primary, secondary or tertiary amine represented by the formula NR²⁹R³⁰R³¹, where each of R²⁹, R³⁰ and R³¹ is independently hydrogen or a hydrocarbyl group of 1 to 32 carbon atoms and wherein at least one of R²⁹, R³⁰ and R³¹ is a hydrocarbyl group of 4 or more carbon atoms or represented by the formula: 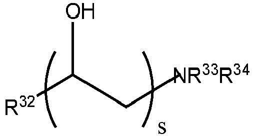 where R³² represents a chain hydrocarbyl group having 10 or more carbon atoms, s is 0 or 1, R³³ and/or R³⁴ represents a hydrogen atom, a hydrocarbyl group, an alkanol group or an alkyl amino group having 2 to 4 carbon atoms, and when s = 0, both R³³ and R³⁴ are not hydrogen atoms or hydrocarbon groups.

Specific examples of suitable amines include monoalkyl (or alkenyl) amines such as tetradecylamine, stearylamine, oleylamine, beef tallow alkylamine, hardened beef tallow alkylamine, and soybean oil alkylamine; dialkyl(or alkenyl)amines such as N-tetradecylmethylamine, N-pentadecylmethylamine, N-hexadecylmethylamine, N-stearylmethylamine, N-oleylmethylamine, N-dococylmethylamine, N-beef tallow alkyl methylamine, N-hardened beef tallow alkyl methylamine, N-soybean oil alkyl methylamine, ditetradecylamine, dipentadecylamine, dihexadecylamine, distearylamine, dioleylamine, didococylamine, bis(2-hexyldecyl)amine, bis(2-octyldodecyl)amine, bis(2-decyltetradecyl)amine, beef tallow dialkylamine, hardened beef tallow dialkylamine, and soybean oil dialkylamine; and trialk(en)ylamines such as tetradecyldimethylamine, hexadecyldimethylamine, octadecyldimethylamine, beef tallow alkyldimethylamine, hardened beef tallow alkyldimethylamine, soybean oil alkyldimethylamine, dioleylmethylamine, tritetradecylamine, tristearylamine, and trioleylamine. Suitable secondary amines have two alkyl (or alkenyl) groups with 14 to 18 carbon atoms.

Examples of the compound containing the hexavalent molybdenum atom include molybdenum trioxides or hydrates thereof (MoO₃·nH₂O), molybdenum acid (H₂MoO₄), alkali metal molybdates (Q₂MoO₄) wherein Q represents an alkali metal such as sodium and potassium, ammonium molybdates {(NH₄)₂MoO₄ or heptamolybdate (NH₄)₆[Mo₇O₂₄].4H₂O}, MoOCl₄, MoO₂Cl₂, MoO₂Br₂, and Mo₂O₃Cl₆. Molybdenum trioxides or hydrates thereof, molybdenum acid, alkali metal molybdates and ammonium molybdates are often suitable because of their availability. In one embodiment, the lubricating composition comprises molybdenum amine compound.

Other organomolybdenum compounds of the invention may be the reaction products of fatty oils, mono-alkylated alkylene diamines and a molybdenum source. Materials of this sort are generally made in two steps, a first step involving the preparation of an aminoamide/glyceride mixture at high temperature, and a second step involving incorporation of the molybdenum.

Examples of fatty oils that may be used include cottonseed oil, groundnut oil, coconut oil, linseed oil, palm kernel oil, olive oil, corn oil, palm oil, castor oil, rapeseed oil (low or high erucic acids), soyabean oil, sunflower oil, herring oil, sardine oil, and tallow. These fatty oils are generally known as glyceryl esters of fatty acids, triacylglycerols or triglycerides.

Examples of some mono-alkylated alkylene diamines that may be used include methylaminopropylamine, methylaminoethylamine, butylaminopropylamine, butylamino-ethylamine, octylaminopropylamine, octylaminoethylamine, dodecylaaminopropylaamine, dodecylaminoethylamine, hexadecylaminopropylamine, hexadecylaminoethylamine, octadecyl-aminopropylamine, octadecylaminoethylamine, isopropyloxypropyl-1,3-diaminopropane, and octyloxypropyl-1,3-diaminopropane. Mono-alkylated alkylene diamines derived from fatty acids may also be used. Examples include N-coco alkyl-1,3-propanediamine (Duomeen^{®}C), N-tall oil alkyl-1,3-propanediamine (Duomeen^{®}T) and N-oleyl-1,3-propanediamine (Duomeen^{®}O), all commercially available from Akzo Nobel.

Sources of molybdenum for incorporation into the fatty oil/diamine complex are generally oxygen-containing molybdenum compounds include, similar to those above, ammonium molybdates, sodium molybdate, molybdenum oxides and mixtures thereof. One suitable molybdenum source comprises molybdenum trioxide (MoOs).

Nitrogen-containing molybdenum compounds which are commercially available include, for example, Sakuralube^{®} 710 available from Adeka which is a molybdenum amine compound, and Molyvan^{®} 855, available from R.T. Vanderbilt.

The nitrogen-containing molybdenum compound may be present in the lubricant composition at 0.005 to 2 wt. % of the composition, or 0.01 to 1.3 wt. %, or 0.02 to 1.0 wt. % of the composition. The molybdenum compound may provide the lubricant composition with 0 to 1000 ppm, or 5 to 1000 ppm, or 10 to 750 ppm 5 ppm to 300 ppm, or 20 ppm to 250 ppm of molybdenum.

### Demulsifiers

Demulsifiers useful herein include trialkyl phosphates, and various polymers and copolymers of ethylene glycol, ethylene oxide, propylene oxide, and mixtures thereof.

### Seal Swell Agents

Seal swell agents useful herein include sulfolene derivatives such as Exxon Necton-37^{™} (FN 1380) and Exxon Mineral Seal Oil^{™} (FN 3200).

### Example Lubricating Composition

An engine lubricant in different embodiments may have a composition as illustrated in Table 1. All additives are expressed on an oil-free basis.

**TABLE 1: Example Lubricating Compositions**

| Additive | Embodiments (wt. %) | | |
|---|---|---|---|
| | A | B | C |
| Polyisobutylene-substituted phenol or derivative thereof | 0.1 to 20 | 0.5 to 10 | 1 to 5 |
| Overbased Sulfonate Detergent | 0 to 9 | 0.3 to 8 | 1 to 5 |
| Phenol-based detergent | 0 to 10 | 0.5 to 7 | 0.75 to 5 |
| (Borated) Dispersant | 0 to 12 | 0.5 to 8 | 1 to 5 |
| Antioxidant | 0 to 13 | 0.1 to 10 | 0.5 to 5 |
| Antiwear Agent | 0 to 15 | 0.1 to 10 | 0.3 to 5 |
| Corrosion Inhibitor | 0 to 2 | 0.1 to 1 | 0.2 to 0.5 |
| Friction Modifier | 0 to 6 | 0.05 to 4 | 0.1 to 2 |
| Viscosity Modifier | 0 to 10 | 0.5 to 8 | 1 to 6 |
| Other Performance Additives | 0 to 10 | 0 to 8 | 0 to 6 |
| Oil of Lubricating Viscosity | Balance to 100 % | | |

### EXAMPLES

### Example 1: Preparation of Isobutylene-substituted phenol:

Phenol is alkylated with a 350 Mn high vinylidene polyisobutylene (obtained from Texas Petroleum Products) under standard conditions using boron trifluoride as a catalyst, as shown in reaction scheme 1 above.

Phenol (271.5 g) and toluene (131.2 g) are first mixed in a nitrogen blanketed vessel followed by the dropwise addition of BF₃ phenol complex (43.7g) over 20 mins. To this mixture, a solution of 350Mw polyisobutylene (404.3 g, Texas Petroleum Products) diluted in toluene (132.3 g) is added dropwise over 4hrs. The addition rate is controlled to maintain a temperature between 13-21°C and never exceeded 28°C.

The reaction mixture is then neutralized slowly with calcium hydroxide (84.0 g) with the temperature being held below 28°C. The neutralized reaction mixture is held for 3hrs following which diatomaceous earth (100 g) is charged to the reaction and the neutralization completed with ammonia hydroxide (10 g). The reaction mixture is filtered and the filtrate heated under vacuum to a temperature of 185°C to remove volatiles by distillation. The resulting material is a 350 Mn polyisobutylene-alkylated phenol.

NMR analysis shows the product to be predominately the para-substituted isomer with <3.0% free polyisobutylene present.

The polyisobutylene substituted phenol described in Example 1 was tested for Acute Ecotox Value for Invertebrate using the OECD Guidelines for the Testing of Chemicals, Test No. 202 Daphnia sp. Acute Immobilisation Test (2004). Samples of alkyl-phenols having C4 and C8 alkyl groups were compared to the Sample of Example 1. A published test value for PDDP using the same test method is also included as a comparison.

| **Example** | **Substance ID** | **Acute Ecotox Value for Invertebrate daphnid (EC50)** |
|---|---|---|
| Comparative Ex. 1 | C4 Alkyl Phenol | 4.7 mg/L |
| Comparative Ex. 2 | C8 Alkyl Phenol | 0.09 mg/L |
| Comparative Ex. 3 | PDDP | 0.037 mg/L* |
| Example 1 | 350 Mn Alkyl Phenol | > 100 mg/L |

| | | |
|---|---|---|
| * Sewell & McKenzie (2005a). ACUTE TOXICITY TO DAPHNIA MAGNA. Testing laboratory: Safepharm Laboratories Limited, Shardlow Business Park, Shardlow, Derbyshire, DE72 2GD, UK. Report no.: 1666/027. Owner company: American Chemistry Council, 1300 Wilson Boulevard, Arlington, VA 22209, USA. Report date: 2005-04-26. | | |

An alkyl phenol prepared in accordance with the present invention shows far lower invertebrate toxicity than the comparative materials.

The singular forms "a," "an," and "the" include plural referents unless the context clearly dictates otherwise.

As used herein, the term "hydrocarbyl substituent" or "hydrocarbyl group" is used in its ordinary sense, which is well-known to those skilled in the art. Specifically, it refers to a group having a carbon atom directly attached to the remainder of the molecule and having predominantly hydrocarbon character. By predominantly hydrocarbon character, it is meant that at least 70% or at least 80% of the atoms in the substituent are hydrogen or carbon. Hydrocarbylene groups are the bivalent equivalents of hydrocarbyl groups, i.e., are attached at each end to two parts of the remainder of the molecule.

Examples of hydrocarbyl groups include:
(i) hydrocarbon substituents, that is, aliphatic (e.g., alkyl or alkenyl), alicyclic (e.g., cycloalkyl, cycloalkenyl) substituents, aryl, and aromatic-, aliphatic-, and alicyclic-substituted aromatic substituents, as well as cyclic substituents wherein the ring is completed through another portion of the molecule (e.g., two substituents together form a ring);
(ii) substituted hydrocarbon substituents, that is, substituents containing non-hydrocarbon groups which, in the context of this invention, do not alter the predominantly hydrocarbon nature of the substituent (e.g., halo (especially chloro and fluoro), hydroxy, alkoxy, mercapto, alkylmercapto, nitro, nitroso, and sulfoxy);
(iii) hetero substituents, that is, substituents which, while having a predominantly hydrocarbon character, may contain other than carbon in a ring or chain otherwise composed of carbon atoms.

Representative alkyl groups useful as hydrocarbyl groups may include at least 1, or at least 2, or at least 3, or at least 4 carbon atoms, and in some embodiments, up to 8, or up to 10, or up to 12, or up to 14, or up to 16, or up to 18 carbon atoms. Illustrative examples include methyl, ethyl, propyl, butyl, pentyl, hexyl, heptyl, octyl, 2-ethylhexyl, nonyl, decyl, undecyl, dodecyl, tridecyl, tetradecyl, hexadecyl, stearyl, icosyl, docosyl, tetracosyl, 2-butyloctyl, 2-butyldecyl, 2-hexyloctyl, 2-hexydecyl, 2-octyldecyl, 2-hexyldodecyl, 2-octyldodecyl, 2-decyltetradecyl, 2-dodecylhexadecyl, 2-hexyldecyloctyldecyl, 2-tetradecyloctyldecyl, 4-methyl-2-pentyl, 2-propylheptyl, monomethyl branched-isostearyl, isomers thereof, mixtures thereof, and the like.

Representative alkenyl groups useful as hydrocarbyl groups include C₂-C₁₈ alkenyl groups, such as ethynyl, 2-propenyl, 1-methylene ethyl, 2-butenyl, 3-butenyl, pentenyl, hexenyl, heptenyl, octenyl, 2-ethylhexenyl, nonenyl, decenyl, undecenyl, dodecenyl, tridecenyl, tetradecenyl, hexadecenyl, isomers thereof, and mixtures thereof.

Representative alicyclic groups useful as hydrocarbyl groups include cyclobutyl, cyclopentyl, and cyclohexyl groups.

Representative aryl groups include phenyl, toluyl, xylyl, cumenyl, mesityl, benzyl, phenethyl, styryl, cinnamyl, benzhydryl, trityl, ethylphenyl, propylphenyl, butylphenyl, pentylphenyl, hexylphenyl, heptylphenyl, octylphenyl, nonylphenyl, decylphenyl, undecylphenyl, dodecylphenyl, benzylphenyl, styrenated phenyl, p-cumylphenyl, α-naphthyl, β-naphthyl groups, and mixtures thereof.

Representative heteroatoms include sulfur, oxygen, nitrogen, and encompass substituents, such as pyridyl, furyl, thienyl and imidazolyl. In general, no more than two, and in one embodiment, no more than one, non-hydrocarbon substituent will be present for every ten carbon atoms in the hydrocarbyl group. In some embodiments, there are no non-hydrocarbon substituents in the hydrocarbyl group.

Numerical values in the specification and claims of this application should be understood to include numerical values which are the same when reduced to the same number of significant figures and numerical values which differ from the stated value by less than the experimental error of conventional measurement technique of the type described in the present application to determine the value.

As used herein, the term "comprising" is inclusive and does not exclude additional, un-recited elements or method steps. However, in each recitation of "comprising" herein, it is intended that the term also encompasses, as alternative embodiments, the phrases "consisting essentially of" and "consisting of," where "consisting of" excludes any element or steps not specified and "consisting essentially of" permits the inclusion of additional un-recited elements or steps that do not materially affect the basic and novel, and essential characteristics of the composition or method under consideration.

## Claims

1. A lubricating composition comprising
(i) an oil of lubricating viscosity and
(ii) a polyisobutylene-substituted phenol having the formula: 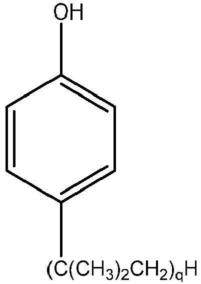 where q is from 3 to 10 and comprises the reaction product of a phenol with a polyisobutylene having a number average molecular weight of 300 to 500.

2. The lubricating composition of claim 1, wherein q is one or more of 4, 5, 6 7, 8, 9, or 10 or mixtures thereof.

3. The lubricating composition of claim 1 or 2, wherein the polyisobutylene has a number average molecular weight of 300 to 400.

4. The lubricating composition of any of claims 1 to 3, wherein the polyisobutylene is prepared in the absence of a chain transfer agent.

5. The lubricating composition of any of claims 1 to 4, wherein the polyisobutylene has a polydispersity of greater than 1.5.

6. The lubricating composition of any of claims 1 to 5, wherein the polyisobutylene-substituted phenol is present in amount from 100 to 1000 ppm.

7. The lubricating composition of any of claims 1 to 6, wherein the oil of lubricating viscosity comprises at least one of an API Group I, II, III, IV, and V base oil.

8. The lubricating composition of any one of claims 1 to 7, wherein the oil of lubricating viscosity is at least 10 wt. % of the lubricating composition, preferably wherein the oil of lubricating viscosity is at least 30 wt. % of the lubricating composition.

9. The lubricating composition of any one of claims 1 to 8, wherein the oil of lubricating viscosity is up to 95 wt. % of the lubricating composition.

10. The lubricating composition of any one of claims 1 to 9, further comprising at least one of the group consisting of detergents, antioxidants, dispersants, antiwear agents, friction modifiers, corrosion inhibitors, and combinations thereof.

11. A method of forming a lubricating composition comprising combining the polyisobutylene-substituted phenol according to any of claims 1-5 with an oil of lubricating viscosity.

12. Use of the lubricating composition of any one of claims 1 to 10 for lubricating a mechanical device.

## Patentansprüche

1. Schmierzusammensetzung, umfassend
(i) ein Öl mit Schmierviskosität und
(ii) ein polyisobutylensubstituiertes Phenol, das die Formel aufweist: 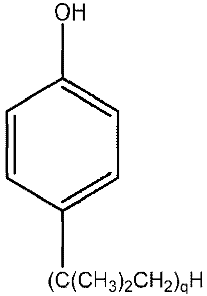 wo q von 3 bis 10 ist und das Reaktionsprodukt eines Phenols mit einem Polyisobutylen, das ein zahlenmittleres Molekulargewicht von 300 bis 500 aufweist, umfasst.

2. Schmierzusammensetzung nach Anspruch 1, wobei q eines oder mehrere von 4, 5, 6, 7, 8, 9 oder 10 oder Mischungen davon ist.

3. Schmierzusammensetzung nach Anspruch 1 oder 2, wobei das Polyisobutylen ein zahlenmittleres Molekulargewicht von 300 bis 400 aufweist.

4. Schmierzusammensetzung nach einem der Ansprüche 1 bis 3, wobei das Polyisobutylen in der Abwesenheit eines Kettenübertragungsmittels hergestellt wird.

5. Schmierzusammensetzung nach einem der Ansprüche 1 bis 4, wobei das Polyisobutylen eine Polydispersität von größer als 1,5 aufweist.

6. Schmierzusammensetzung nach einem der Ansprüche 1 bis 5, wobei das polyisobutylensubstituierte Phenol in einer Menge von 100 bis 1000 ppm vorhanden ist.

7. Schmierzusammensetzung nach einem der Ansprüche 1 bis 6, wobei das Öl mit Schmierviskosität mindestens eines von einem Basisöl von API-Gruppe I, II, III, IV und V umfasst.

8. Schmierzusammensetzung nach einem der Ansprüche 1 bis 7, wobei das Öl mit Schmierviskosität mindestens 10 Gew. % der Schmierzusammensetzung ausmacht, wobei vorzugsweise das Öl mit Schmierviskosität mindestens 30 Gew. % der Schmierzusammensetzung ausmacht.

9. Schmierzusammensetzung nach einem der Ansprüche 1 bis 8, wobei das Öl mit Schmierviskosität bis zu 95 Gew.-% der Schmierzusammensetzung ausmacht.

10. Schmierzusammensetzung nach einem der Ansprüche 1 bis 9, ferner umfassend mindestens eines der Gruppe, bestehend aus Detergenzien, Antioxidationsmitteln, Dispergiermitteln, Verschleißschutzmitteln, Reibungsmodifikatoren, Korrosionsinhibitoren und Kombinationen davon.

11. Verfahren zum Ausbilden einer Schmierzusammensetzung, umfassend ein Kombinieren des polyisobutylensubstituierten Phenols nach einem der Ansprüche 1 bis 5 mit einem Öl mit Schmierviskosität.

12. Verwendung der Schmierzusammensetzung nach einem der Ansprüche 1 bis 10 zum Schmieren einer mechanischen Vorrichtung.

## Revendications

1. Composition lubrifiante comprenant
(i) une huile de viscosité lubrifiante et
(ii) un phénol substitué par un polyisobutylène ayant la formule : 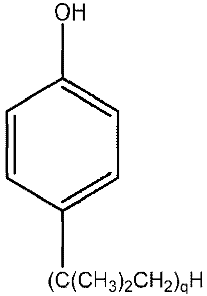 où q est compris entre 3 et 10 et comprend le produit de réaction d'un phénol avec un polyisobutylène ayant un poids moléculaire moyen en nombre compris entre 300 et 500.

2. Composition lubrifiante selon la revendication 1, dans laquelle q est un ou plusieurs des 4, 5, 6, 7, 8, 9, ou 10 ou des mélanges de ceux-ci.

3. Composition lubrifiante selon la revendication 1 ou 2, dans laquelle le polyisobutylène a un poids moléculaire moyen en nombre de 300 à 400.

4. Composition lubrifiante selon l'une quelconque des revendications 1 à 3, dans laquelle le polyisobutylène est préparé en l'absence d'agent de transfert en chaîne.

5. Composition lubrifiante selon l'une quelconque des revendications 1 à 4, dans laquelle le polyisobutylène a une polydispersion supérieure à 1,5.

6. Composition lubrifiante selon l'une des revendications 1 à 5, dans laquelle le phénol substitué par un polyisobutylène est présent en quantité comprise entre 100 et 1000 ppm.

7. Composition lubrifiante selon l'une quelconque des revendications 1 à 6, dans laquelle l'huile de viscosité lubrifiante comprend au moins une huile de base API des groupes I, II, III, IV et V.

8. Composition lubrifiante selon l'une des revendications 1 à 7, dans laquelle l'huile de viscosité lubrifiante est au moins de 10 % en poids de la composition lubrifiante, de préférence dans laquelle l'huile de viscosité lubrifiante est d'au moins 30 % en poids de la composition lubrifiante.

9. Composition lubrifiante selon l'une quelconque des revendications 1 à 8, dans laquelle l'huile de viscosité lubrifiante représente au moins 95 % en poids de la composition lubrifiante.

10. Composition lubrifiante selon l'une quelconque des revendications 1 à 9, comprenant en outre au moins l'un des groupes constitué de détergents, antioxydants, dispersants, agents antiusure, modificateurs de frottement, inhibiteurs de corrosion et leurs combinaisons.

11. Procédé de fabrication d'une composition lubrifiante consistant à combiner le phénol substitué par un polyisobutylène selon l'une des revendications 1 à 5 avec une huile de viscosité lubrifiante.

12. Utilisation de la composition lubrifiante selon l'une quelconque des revendications 1 à 10 pour lubrifier un dispositif mécanique.
